(19) Europäisches Patentamt
European Patent Office
Office européen des brevets

(11) EP 4 714 958 A1

(12) **EUROPEAN PATENT APPLICATION**
published in accordance with Art. 153(4) EPC

(43) Date of publication:
**25.03.2026 Bulletin 2026/13**

(21) Application number: **24810270.9**

(22) Date of filing: **14.05.2024**

(51) International Patent Classification (IPC):
***C07D 519/00*** *(2006.01)* ***A61K 31/409*** *(2006.01)*
***A61K 41/00*** *(2020.01)* ***A61K 39/395*** *(2006.01)*
***A61P 35/00*** *(2006.01)*

(52) Cooperative Patent Classification (CPC):
**A61K 31/409; A61K 39/395; A61K 41/00; A61K 45/06; A61P 35/00; A61P 35/04; C07D 519/00**

(86) International application number:
**PCT/CN2024/093175**

(87) International publication number:
**WO 2024/240023 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.05.2023 CN 202310574518**

(71) Applicant: **Shanghai Guangsheng Biopharmaceutical Co., Ltd**
**Shanghai 201318 (CN)**

(72) Inventors:
- **ZHANG, Shaoliang**
**Shanghai 201318 (CN)**
- **ZHANG, Hao**
**Shanghai 201318 (CN)**

(74) Representative: **Monteiro Alves, Inês**
**Alameda Dos Oceanos, Nº 41K-21**
**Parque das Nações**
**1990-207 Lisboa (PT)**

# (54) COMBINED THERAPY OF PHOTODYNAMIC THERAPY AND IMMUNE CHECKPOINT INHIBITOR

(57) Provided are a combined therapy of photodynamic therapy and immunological therapy, comprising the combined administration of a porphyrin dimer salt-based photosensitizer and an immune checkpoint inhibitor, and the use thereof in treatment of recurrent and/or metastatic cancers, in particular esophageal cancer.

EP 4 714 958 A1

## Description

### Technical Field

The present application relates to a combined therapy of photodynamic therapy and immune checkpoint inhibitor, in particular in treatment of systemic or local recurrent and/or metastatic cancers, in particular esophageal cancer.

### Background

Esophageal Cancer (EC) is the ninth most common cancer worldwide, the sixth most common cancer in China. Radical treatment of EC includes surgery, radical chemoradiotherapy, drug therapy, etc. Most patients have already developed into late-stage disease at the time of diagnosis. Even after radical surgery or chemoradiotherapy, the rate of recurrence of EC is still high, while the 5-year survival rate for patients with distant metastatic disease is only 4.8%.

The standard first-line treatment regimen for EC is chemotherapy based on cisplatin +5-Fu or paclitaxel. But after first-line treatment advances, systemic treatment options are limited and only effective for a short period. While radiotherapy is usually applied to primary or local recurrent tumors, complications are high (e.g. radiation pneumonitis, esophagitis, fistulae and bleeding), the prognosis of patients is worse, the quality of life is affected, and local recurrence after radiotherapy is a major problem for patients with esophageal cancer. In recent years, immunological therapy has become the second-line treatment choice for patients. Clinical studies have shown that second-line immunological therapy is effective in treatment of esophageal squamous cell carcinoma (ESCC), with a remission rate of 16.7%. However, the major difficulties faced by immunological therapy alone are manifold, including problems of large individual variation, insufficient immunogenicity, poor intervention of locally primary or recurrent lesions, etc. Therefore, there is an urgent need to develop a novel local control therapy with a high remission rate for local esophageal cancer and distant metastatic lesions, combining with other treatment regimens may be an effective way to increase the remission rate.

Photodynamic therapy (PDT) is a new approach to disease treatment that utilizes the photodynamic effect of photosensitizers, and is currently used primarily for treatment and diagnosis of malignant tumors and certain precancerous and benign lesions. After photosensitizer is injected into the body, relatively high-concentration accumulation can be formed in tumor tissue in a period of time, and when irradiated with light of a specific wavelength, a strong photodynamic effect occurs, resulting in significant destruction of the diseased tissue. PDT has certain selectivity on target tissues and good killing controllability, so that the damage of normal tissues can be reduced as much as possible under the condition of ensuring tumor inactivation, and the PDT is a local treatment method with micro-invasiveness, low toxicity and non-thermogenesis, thus possessing wide application prospect.

The present invention provides a combined therapy of photodynamic therapy based on a porphyrin dimer salt-based photosensitizer and an immune checkpoint inhibitor in treatment of local recurrent cancer, in particular oesophageal cancer.

### Summary

In one aspect, the application provides a photosensitizer suitable for use in treating cancer in an individual, the photosensitizer comprises a porphyrin dimer salt having a structure represented by formula (I), formula (II), or formula (III):

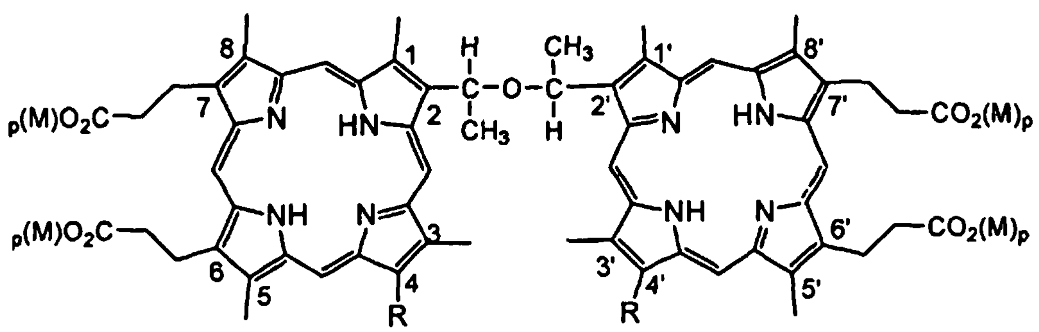

formula (I),

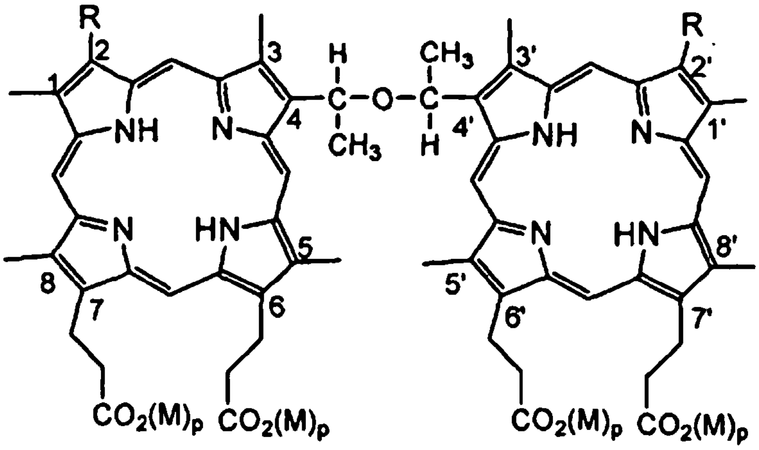

formula (II), or

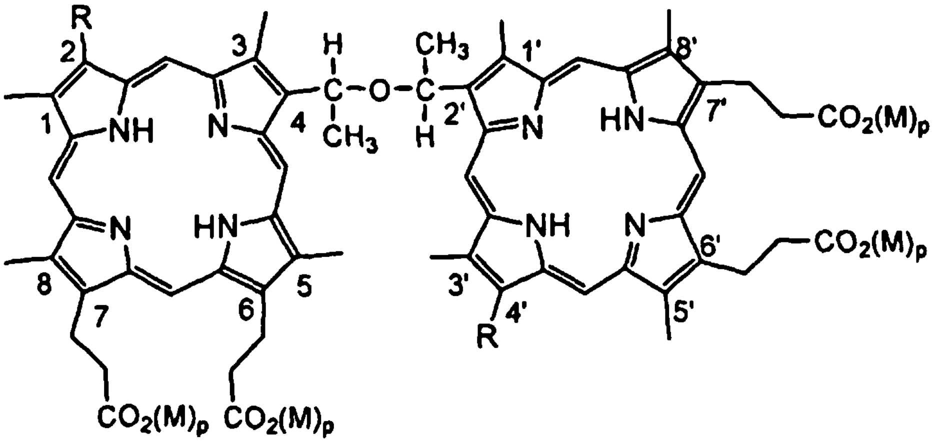

formula (III)

Wherein,

R is independently selected from $C_{1-6}$ alkyl, $C_{2-6}$ alkenyl, $C_{2-6}$ alkynyl, $C_{1-6}$ alkoxy and $C_{1-6}$ acyl, optionally substituted with one or more substituents selected from halogen and hydroxy,
M is an alkali metal, an alkaline earth metal or $NH_4^+$,
p is the reciprocal of the valence number of M,
and solvates, especially hydrates, thereof.

In some embodiments, wherein R is independently selected from $C_{1-6}$ alkyl, $C_{2-6}$ alkenyl, $C_{2-6}$ alkynyl, and $C_{1-6}$ acyl optionally substituted with one or more hydroxy groups, M is an alkali metal or $NH_4$+, and p is 1, and solvates, especially hydrates, thereof.

In some embodiments, wherein R is independently selected from -CH = $CH_2$, - CH(OH)$CH_3$ and -$COCH_3$, M is an alkali metal or $NH_4$+, and p is 1, and solvates, especially hydrates, thereof.

In some embodiments, wherein R is independently selected from -CH = $CH_2$ and - CH(OH)$CH_3$, M is an alkali metal or $NH_4$+, and p is 1, and solvates, especially hydrates, thereof.

In some embodiments, wherein M is an alkali metal, such as Na or K.

In some embodiments, wherein the ether-bond bound porphyrin dimer salt is selected from the group consisting of:

bis [1- [6, 7-sodium dipropionate-1, 3, 5, 8-tetramethyl-4-vinyl-2-porphine] ethyl] ether (DVDMS-1),
bis [1- [6, 7-sodium dipropionate-1, 3, 5, 8-tetramethyl-2-vinyl-4-porphine] ethyl] ether (DVDMS-2),
1- [6, 7-sodium dipropionate-1, 3, 5, 8-tetramethyl-2-vinyl-4-porphine] ethyl 1- [6', 7' -sodium dipropionate-1', 3', 5', 8'

-tetramethyl-4-vinyl-2-porphine] ethyl ether (DVDMS-3),
bis [1- [6, 7-potassium dipropionate-1, 3, 5, 8-tetramethyl-4-vinyl-2-porphine] ethyl] ether,
bis [1- [6, 7-potassium dipropionate-1, 3, 5, 8-tetramethyl-2-vinyl-4-porphine] ethyl] ether,
1- [6, 7-potassium dipropionate-1, 3, 5, 8-tetramethyl-2-vinyl-4-porphine] ethyl 1- [6', 7' - potassium dipropionate-1', 3', 5', 8' -tetramethyl-4-vinyl-2-porphine] ethyl ether,
bis [1- [6, 7-ammonium dipropionate-1, 3, 5, 8-tetramethyl-4-vinyl-2-porphine] ethyl] ether,
bis [1- [6, 7-ammonium dipropionate-1, 3, 5, 8-tetramethyl-2-vinyl-4-porphine] ethyl] ether,
and
1- [6, 7-ammonium dipropionate-1, 3, 5, 8-tetramethyl-2-vinyl-4-porphine] ethyl 1- [6', 7' - ammonium dipropionate-1', 3', 5', 8' -tetramethyl-4-vinyl-2-porphine] ethyl ether.

In some embodiments, the porphyrin dimer salt is selected from the group consisting of: DVDMS-1, DVDMS-2, DVDMS-3 and DVDMS-M (mixture of DVDMS-1, DVDMS-2, DVDMS-3).

In another aspect, the application provides a method of treating cancer in an individual, comprising administering to the individual a therapeutically effective amount of a photosensitizer comprising a porphyrin dimer salt as disclosed herein, in combination with an immune checkpoint inhibitor.

In some embodiments, the cancer is esophageal cancer. In some embodiments, the esophageal cancer may be pathologically typed and selected from esophageal squamous cell carcinoma (ESCC), adenocarcinoma, mucoepidermoid carcinoma, small cell carcinoma, large cell carcinoma, neuroendocrine tumor, adenosquamous carcinoma, undifferentiated carcinoma, optionally esophageal squamous cell carcinoma (ESCC) or adenocarcinoma. In some embodiments, the esophageal cancer is advanced esophageal cancer, or alternatively, locally advanced esophageal cancer. In some embodiments, the esophageal cancer is recurrent esophageal cancer (optionally, local recurrent esophageal cancer) and/or metastatic esophageal cancer (optionally, distant metastatic esophageal cancer).

In some embodiments, the individual has received radiotherapy and/or chemotherapy.

In some embodiments, the individual has been treated for a primary lesion and/or a metastatic lesion.

In some embodiments, the esophageal cancer exhibits resistance to at least one chemotherapy selected from the group consisting of: taxane-based antitumor chemotherapy drug, fluorouracil-based drug, and platinum-based drug. In some embodiments, the taxane-based antitumor chemotherapy drug is selected from the group consisting of: paclitaxel, docetaxel. In some embodiments, the fluorouracil-based drug is selected from the group consisting of: 5-fluorouracil, tegafur, capecitabine. In some embodiments, the platinum-based drug is selected from the group consisting of: cisplatin, carboplatin, oxaliplatin.

In some embodiments, the esophageal cancer exhibits immunotherapeutic resistance.

In some embodiments, the porphyrin dimer salt is administered in the range of 0.01-5mg/kg body weight. In some embodiments, the individual is photodynamic treated using a laser 24-48 hours after the porphyrin dimer salt administration. In some embodiments, the laser has a wavelength of 631 $\pm$ 3nm, such as 630nm, 631nm, or 632 nm. In some embodiments, the laser dosage is 90-300J/cm$^2$.

In some embodiments, the immune checkpoint inhibitor is administered to the individual after a period of time following the laser administration. In some embodiments, the immune checkpoint inhibitor is administered to the individual 20 to 50 hours, preferably 24 to 48 hours after the laser administration.

In some embodiments, the immune checkpoint inhibitor is selected from the group consisting of: PD-1 antagonist (PD-1 antibody), PD-L1 antagonist (PD-L1 antibody). In some embodiments, the PD-1 antibody is selected from the group consisting of Pembrolizumab, Nivolumab, Cemiplimab, Toripalimab, Sintilimab, Camrelizumab, Tislelizumab, Penpulimab, Sepalimab, Serplulimab, and Putelimumab. In some embodiments, the PD-L1 antibody is selected from the group consisting of Atezolizumab, Avelumab, Durvalumab, Envafolimab and Sugemalimab.

In some embodiments, the porphyrin dimer salt is sinoporphyrin sodium (DVDMS) that is a sodium porphyrin dimer bound by an ether linkage. In some embodiments, the sinoporphyrin sodium has the structure of formula (I), formula (II), or formula (III) above. In some embodiments, the porphyrin dimer salt is DVDMS-1, DVDMS-2, DVDMS-3, or DVDMS-M (i.e., a mixture of DVDMS-1, DVDMS-2, DVDMS-3), and wherein the immune checkpoint inhibitor is Pembrolizumab.

In yet another aspect, the application also provides the use of a photosensitizer as disclosed herein in the preparation of drug for the treatment of cancer in an individual, wherein the treatment comprises the combined therapy of a photosensitizer and an immune checkpoint inhibitor. In some embodiments, wherein the cancer is esophageal cancer. In some embodiments, the application also provides the use of a photosensitizer as disclosed herein and an immune checkpoint inhibitor in the preparation of drug for the treatment of cancer in an individual. In some embodiments, the medicament is used to practice the disclosed methods of treating cancer. In some embodiments, the photosensitizer and immune checkpoint inhibitor as disclosed herein are prepared in a form suitable for use in the methods of treating cancer as disclosed herein. In yet another aspect, the application also provides a pharmaceutical composition. In some embodiments, the pharmaceutical composition comprises a photosensitizer as disclosed herein. In some embodiments, the pharmaceutical composition is used to practice the disclosed methods of treating cancer.

In yet another aspect, the application also provides a kit comprising a photosensitizer as disclosed herein. In some embodiments, the kit comprises a photosensitizer as disclosed herein. In some embodiments, the kit is used to practice the disclosed methods of treating cancer. In some embodiments, the kit further comprises an immune checkpoint inhibitor.

Unwilling to be bound by theory, in the combined therapy of photodynamic therapy based on a porphyrin dimer salt-based photosensitizer and immunological therapy described in this application, the photodynamic therapy has the characteristics of neoadjuvant therapy such as intervention, combination and the like in the immunological therapy, firstly, the immunogenicity in the body of a patient is preferentially activated through the photodynamic therapy, so that the effects of providing a higher immune system basis for subsequent immunological therapy means or medicines, strengthening the cell activity of immunological therapy and the like are achieved, and the treatment effectiveness of the combined therapy is improved. The combined therapy described in this application is suitable for treating various cancers, particularly esophageal cancer, and especially provides a novel systemic treatment method for advanced esophageal cancer with poor effect of the previous treatment, has a comprehensive inhibition effect on primary and even systemically metastatic tumors, is beneficial for effectively eliminating cancer cells, and reduces the probability of recurrence and/or metastasis of the cancer cells. The combined therapy described in this application can be further combined with other treatment methods, for example, after surgical treatment of lesions, the combined therapy described in this application is used for secondary intervention and elimination treatment, so as to enhance the clearance rate of cancer cells, reduce the recurrence rate of tumors and improve the survival rate of patients.

## Detailed Description

The following description of the application is intended only to illustrate various embodiments of the application. Therefore, the specific modifications discussed should not be construed as limitations on the scope of the application. It will be apparent to those skilled in the art that various equivalents, changes, and modifications may be made without departing from the scope of the application, and it is to be understood that such equivalent embodiments are to be included herein. All references, including publications, patents, and patent applications, cited herein are hereby incorporated by reference in their entirety.

The present application provides a combined therapy of photodynamic therapy based on a porphyrin dimer salt-based photosensitizer and immunological therapy in the treatment of cancer in an individual, comprising administering to the individual a therapeutically effective amount of a photosensitizer comprising a porphyrin dimer salt, in combination with an immune checkpoint inhibitor. The combined therapy is suitable for the treatment of various types of cancer, such as systemic or local recurrent and/or metastatic cancers, in particular esophageal cancer.

### Esophageal cancer

As used herein, the terms "cancer," "malignancy," "tumor," and "carcinoma" are used interchangeably herein to refer to a cell that exhibits relatively abnormal, uncontrolled, and/or autonomous growth such that the cell exhibits an abnormal growth phenotype characterized by significant deregulation of cell proliferation. As used herein, the terms "cancer," "malignancy," "tumor," and "carcinoma" may include primary, or metastatic.

The term "esophageal cancer" refers to a tumor that occurs in esophageal epithelial tissue. Classified by pathological typing, esophageal cancers include, but are not limited to: esophageal squamous cell carcinoma (ESCC), adenocarcinoma, mucoepidermoid carcinoma, small cell carcinoma, large cell carcinoma, neuroendocrine tumor, adenosquamous carcinoma, or undifferentiated carcinoma. In some embodiments, the esophageal cancer is esophageal squamous cell carcinoma (ESCC) or adenocarcinoma.

In some embodiments, esophageal cancer described in this application comprises advanced esophageal cancer. In some embodiments, the esophageal cancer is locally advanced esophageal cancer.

According to the cancer invasion range, the stage of the esophageal cancer is judged according to the TNM stage standard. Generally, in oncology, the esophageal cancer of TNM stages I and II are judged as early esophageal cancer, and TNM stages III and IV are judged as advanced esophageal cancer or locally advanced esophageal cancer. The term "locally advanced esophageal cancer" generally refers to a condition in which the esophagus becomes cancerous, invades the esophagus and surrounding tissues, but does not invade vital organs, nor does it metastasize remotely. In some embodiments, the locally advanced esophageal cancer includes esophageal cancer that fails first-line therapy, second-line therapy, and even third-line therapy, e.g., with the following characteristics: lymph and distant metastases exist, the basic condition of the body of a patient is poor, and a treatment method aiming at radical treatment cannot be achieved.

The term "first-line therapy" refers to the first round of treatment after diagnosis, with the best efficacy and minimal side effects, also referred to as primary treatment or therapy, with the goal of effectively inhibiting tumor progression, where possible. The term "second-line therapy" refers to the situation where, after first-line treatment, the patient experiences

tumor progression again and develops resistance to the first-line treatment regimen, necessitating a switch to a regimen with a different anti-cancer mechanism. The term "third-line therapy" refers to therapy that is replaced with another regimen after failure of the second-line therapy, usually with fewer available drugs and effective treatment options.

In some embodiments, the esophageal cancer is recurrent esophageal cancer. In some embodiments, the esophageal cancer is a local recurrent esophageal cancer. The term "recurrent esophageal cancer" refers to the recurrence of the same type of tumor as the primary esophageal tumor after the esophageal tumor at the primary site has been treated to regress. In some embodiments, the recurrent esophageal cancer is a systemically recurrent esophageal cancer or a local recurrent esophageal cancer.

In some embodiments, the esophageal cancer is metastatic esophageal cancer. In some embodiments, the esophageal cancer is a distant metastatic esophageal cancer. The term "metastatic esophageal cancer" refers to a situation in which in addition to the primary esophageal lesion, the cancer metastasizes to adjacent or distant organs via lymph or blood. The term "distant metastatic esophageal cancer" refers to a metastasis that has spread to areas distant from the primary esophageal cancer lesion. For example, esophageal cancer that originally existed in the lower esophageal segment, if it is metastasized to the lymph node above the clavicle, is considered to be a distant metastatic esophageal cancer that is metastasized to a distant lymph node. For another example, if the cancer is originally esophageal cancer in the neck and metastasizes to a lymph node in the neck, it is considered to be metastasis to a proximal lymph node.

In some embodiments, the recurrent or metastatic esophageal cancer has caused esophageal obstruction, preventing the patient from independently eating or drinking water. One of the current primary treatment methods for this kind of esophageal cancer is to open the esophageal passage, improve the eating difficulty of patients and improve the nutritional requirements of the patients, thereby being beneficial to strengthening the palliative treatment effect. At present, except for palliative radiotherapy and chemotherapy, the primary treatment methods of opening the esophageal passage include physical treatment methods such as esophageal stent ablation, surgical electrocautery resection, balloon dilatation, etc., but the methods have significant defects, only can provide temporary physical dilatation, cannot solve the recurrence and blockage of esophageal cancer in the esophagus, and cannot truly eliminate cancer cells in the esophagus. The combined therapy of photodynamic therapy based on a porphyrin dimer salt-based photosensitizer and immunological therapy as described in the present application unexpectedly and effectively solves the above mentioned problems.

On the one hand, by virtue of the massive accumulation of photosensitizers within the tumor, tumor cells can be killed by photodynamic action under illumination of a specific wavelength. On the other hand, after the photodynamic therapy destroys tumor cells, a large amount of tumor antigens can be released, so that the immune response in a patient body can be preferentially activated, and the basic immune system is improved. The inventors of the application find that when the subsequent immunological therapy or medicines are matched, the activity of immune cells can be obviously enhanced, and the treatment effectiveness of patients is improved, so that the problem of esophageal passage blockage caused by esophageal cancer is solved, the recurrence probability of tumor is fundamentally inhibited, the self-feeding problem of patients is solved, the life quality and nutritional support of the patients are greatly improved, a good treatment foundation is laid for the subsequent palliative treatment, which is beneficial for prolonging the survival of patients.

In some embodiments, esophageal cancer described in the present application further comprises esophageal cancer that exhibits resistance to at least one of immunological therapy and chemotherapy. In the present application, "drug resistance" means that after a certain period of treatment, the patient has developed a resistance mechanism effect against the therapeutic agent (e.g., immunological therapy and/or chemotherapy), and is resistant or unresponsive, and cannot benefit the therapeutic effect of the drug any more. For example, the number of tumor cells still increases even when treated with a therapeutic agent. The drug resistance may be developed prior to treatment (i.e., primary) or developed after treatment (i.e., acquired, or recurrent). Unwilling to be bound by theory, the porphyrin dimer salt described in the present application belongs to porphyrin-based photosensitizer, and porphyrin is an essential substance in the synthesis process of human erythrocytes, and a large amount of porphyrin-based substances exist in a human body, so that after the porphyrin-based photosensitizer runs in the body, the immune system is insensitive to the porphyrin-based photosensitizer, and a drug resistance mechanism is lacked, so that the porphyrin-based photosensitizer described in the present application can be used for single, multiple or even high-frequency treatment without losing the effectiveness of tumor treatment. Therefore, the combined therapy of the application is not easy to generate drug resistance in the body of a patient and can still function stably after a certain period of use.

In some embodiments, the esophageal cancer described in the present application exhibits resistance to one or more chemotherapeutic agents.

"Chemotherapy" as used herein is biological (macromolecular) or chemical (small molecule) compounds that can be used to treat cancer. The types of chemotherapeutic drugs include, but are not limited to: histone deacetylase inhibitors (HDACI), alkylating agents, antimetabolites, alkaloids, cytotoxic/anticancer antibiotics, topoisomerase inhibitors, tubulin inhibitors, proteins, antibodies, kinase inhibitors, etc. Chemotherapeutic drugs include compounds for targeted therapy and compounds for non-targeted conventional chemotherapy.

In some embodiments, the esophageal cancer exhibits resistance to at least one chemotherapy, which may be any conventional chemotherapy or clinically standard chemotherapy known in the art, including but not limited to the following drugs or combinations thereof: taxane-based antitumor chemotherapy drug, fluorouracil-based drug, platinum-based drug, antifolate-based drug, and topoisomerase inhibitor. Examples of taxane-based antitumor chemotherapy drug include, for example, paclitaxel-based compounds and docetaxel-based compounds. Examples of fluorouracil-based drug include, for example, 5-fluorouracil, tegafur (i.e., a combination of tegafur, gimeracil, and oteracis), capecitabine, etc. Examples of platinum-based drug include, for example, cisplatin, carboplatin, oxaliplatin, nedaplatin, etc. Examples of antifolate-based drug include methotrexate, pemetrexed, etc. Examples of topoisomerase inhibitor include irinotecan.

In some embodiments, the esophageal cancer includes, but is not limited to, exhibiting resistance to at least one chemotherapeutic drug regimen selected from the group consisting of: common chemotherapy regimens for advanced ESCC including cisplatin in combination with fluorouracil or paclitaxel in combination with platinum-based drug, etc.; common chemotherapy regimens for advanced esophagogastric junction adenocarcinoma including cisplatin or oxaliplatin in combination with fluorouracil-based drug, etc.; the first-line treatment regimens aiming at esophageal cancer patients with good physical conditions including a three-medicine combination regimen of taxane-based drug in combination with platinum-base drug and fluorouracil-based drug.

In some embodiments, the esophageal cancer described in the present application exhibits resistance to one or more immunotherapies.

Herein, "immunological therapy" refers to a treatment method that utilizes the patient's own immune cells to eliminate cancer by enhancing and activating their function. Examples of immunological therapy include, but are not limited to: molecular targeted therapy; immune checkpoint inhibitors (e.g., PD-1/L1 inhibitors and CTLA-4 inhibitors); adoptive immune cell therapy (e.g., CAR-T, TIL, NK, CIK/DC-CIK); cytokine therapy; or a tumor vaccine.

In some embodiments, the esophageal cancer described herein exhibits resistance to immune checkpoint inhibitors.

The term "immune checkpoint inhibitor" as used herein refers to a drug capable of targeting an immune checkpoint molecule, relieving immune cells from the inhibition of tumor cells, causing immune cells to reactivate and recognize/kill cancer cells.

In some embodiments, the immune checkpoint inhibitor is PD-1 antagonist (PD-1 antibody) or PD-L1 antagonist (PD-L1 antibody).

In some embodiments, the PD-1 antibody includes, but is not limited to: Pembrolizumab, Nivolumab, Cemiplimab, Toripalimab, Sintilimab, Camrelizumab, Tislelizumab, Penpulimab, Sepalimab, Serplulimab and Putelimumab.

In some embodiments, the PD-L1 antibodies include, but are not limited to: Atezolizumab, Avelumab, Durvalumab, Envafolimab and Sugemalimab.

In some embodiments, the esophageal cancer described in the present application exhibits resistance to at least one immunological therapy.

In some embodiments, the esophageal cancer of the present application exhibits resistance to at least one of the PD-L1 antibody or PD-1 antibody.

In some embodiments, the resistance to immunological therapy may be any conventional immunological therapy regimen or clinically standard immunological therapy regimen known in the art, including, but not limited to the following drug regimens: the second-line therapy regimen for advanced esophageal squamous cell carcinoma (ESCC) patients who have failed the first-line chemotherapy, e.g., including Camrelizumab or Tislelizumab; the second-line treatment regimen for ESCC patients with PD-L1 CPS >10 who have failed the first-line chemotherapy, e.g., including Pembrolizumab monotherapy; the third-line and subsequent treatment regimen for patients with oesophageal-gastric junction adenocarcinoma who have failed at least the second-line chemotherapy, e.g., including Nivolumab; the third-line and subsequent treatment regimen for HER-2 positive advanced esophageal-gastric junction cancer, e.g.,including HER-2 targeting antibody drug conjugate Disitamab Vedotin; the third-line and subsequent treatment regimen for patients with advanced esophageal-gastric junction cancer, e.g., including anti-angiogenic targeted drug Apatinib; the second-line and subsequent treatment regimen for advanced ESCC patients, e.g., including Anlotinib or Apatinib.

In some embodiments, the esophageal cancer described in the present application is esophageal cancer that exhibits resistance to a combined therapy of immunological therapy and chemotherapy.

In some embodiments, the esophageal cancer includes, but is not limited to, cases exhibiting resistance to the following combined therapy regimen: the first-line treatment regimen for HER-2 positive patients with advanced oesophageal-gastric junction cancer, including Trastuzumab in combination with cisplatin and fluorouracil-based drug.

In some embodiments, the individual diagnosed with esophageal cancer (e.g., exhibiting drug resistance) has previously received radiotherapy and/or chemotherapy.

In some embodiments, the individual diagnosed with esophageal cancer (e.g., exhibiting drug resistance) has been treated for a primary or metastatic lesion.

Photosensitizer

The photosensitizer herein comprises porphyrin dimer salt. The porphyrin dimer salt refers to a compound structure having two porphyrin structures connected by a covalent bond or a linker.

In some embodiments, the porphyrin dimer salt has a structure represented by formula (I), formula (II), or formula (III) below:

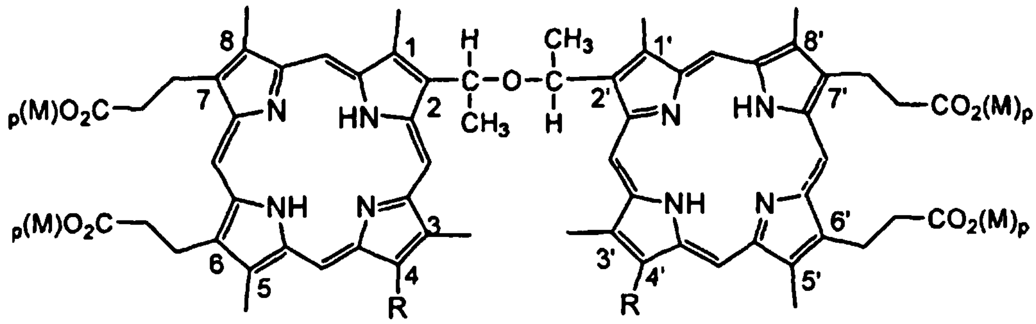

formula (I),

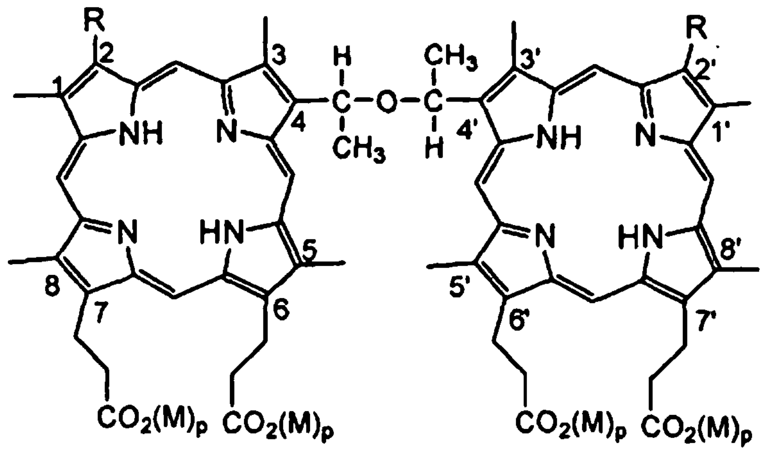

formula (II), or

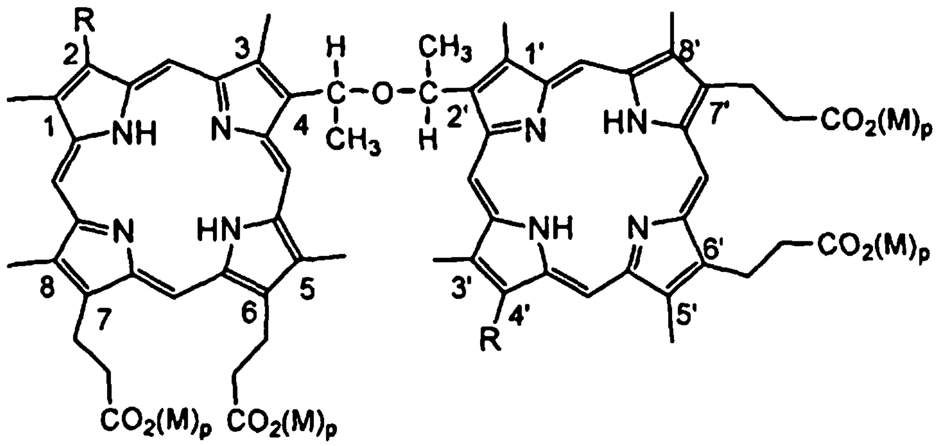

formula (III)

Wherein,

R is independently selected from $C_{1-6}$ alkyl, $C_{2-6}$ alkenyl, $C_{2-6}$ alkynyl, $C_{1-6}$ alkoxy and $C_{1-6}$ acyl, optionally substituted with one or more substituents selected from halogen and hydroxy,
M is an alkali metal, an alkaline earth metal or $NH_4^+$,
p is the reciprocal of the valence number of M,
and solvates, especially hydrates, thereof.

In the present application, "$C_{1-6}$ alkyl" means a branched or straight-chain monovalent saturated hydrocarbon group having 1 to 6 carbon atoms. $C_{1-4}$ alkyl is preferred. Examples include, but are not limited to, methyl, ethyl, n-propyl, isopropyl, n-butyl, sec-butyl, tert-butyl, n-pentyl, isopentyl, neopentyl, n-hexyl, etc.
In the present application, "$C_{2-6}$ alkenyl" means a branched or straight-chain monovalent unsaturated hydrocarbon group having 2 to 6 carbon atoms, which contains one or more double bonds. $C_{2-4}$ alkenyl is preferred. Examples include, but are not limited to, vinyl, prop-1-enyl, allyl, but-1-enyl, pent-1-enyl, pent-1,4-dienyl, etc.
In the present application, "$C_{2-6}$ alkynyl" means a branched or straight-chain monovalent unsaturated hydrocarbon group having 2 to 6 carbon atoms, which contains one or more triple bonds. $C_{2-4}$ alkynyl is preferred. Examples include, but are not limited to, ethynyl, prop-1-ynyl, prop-2-ynyl, but-1-ynyl, pent-1-ynyl, pent-1,3-diynyl, etc.
In the present application, "$C_{1-6}$ alkoxy" means the group R'-O-, wherein R' is $C_{1-6}$ alkyl as defined above. $C_{1-4}$ alkoxy is preferred. Examples include, but are not limited to, methoxy, ethoxy, n-propoxy, isopropoxy, n-butoxy, sec-butoxy, tert-butoxy, n-pentoxy, isopentoxy, neopentoxy, and n-hexoxy, etc.
In the present application, "$C_{1-6}$ acyl" means a group R'-C(O)-, wherein R' is H or a branched or straight-chain monovalent saturated hydrocarbon group having 1 to 5 carbon atoms. $C_{1-4}$ acyl is preferred. Examples include, but are not limited to, formyl, acetyl, propionyl, butyryl, pentanoyl, hexanoyl, etc.
In the present application, "optional" or "optionally" means that the subsequently described event or circumstance may but need not occur, and that the description includes instances where the event or circumstance occurs and instances where it does not. For example, "$C_{1-6}$ alkyl ......, optionally substituted with one or more substituents selected from halogen and hydroxy" means $C_{1-6}$ alkyl that can be, but not must be, substituted with one or more halogen and/or hydroxy, and this description includes unsubstituted $C_{1-6}$ alkyl and $C_{1-6}$ alkyl substituted with one or more halogen and/or hydroxy.
In the present application, "halogen" means fluorine, chlorine, bromine and iodine, preferably fluorine, chlorine and bromine, more preferably fluorine and chlorine.
In the present application, "hydroxy" means the group -OH.
In the present application, "valence number" means the number of shared electron pairs formed by one atom of a chemical element. The valence number of an ion is equal to its charge number. For example, the valence number of alkali metals and $NH_4^+$ is 1, and the valence number of alkaline earth metals is 2.
In the present application, "solvate" means a solvent combination form in which solvent molecules and the ether bond-bound porphyrin dimer salt described herein are combined together by mutual attraction. If the solvent is water, the solvate formed is a hydrate, such as a monohydrate or a dihydrate; if the solvent is alcohol, the solvate formed is an alcoholate, e.g. the addition form with methanol or ethanol.
In some embodiments, R is independently selected from $C_{1-6}$ alkyl, $C_{2-6}$ alkenyl, $C_{2-6}$ alkynyl, and $C_{1-6}$ acyl optionally substituted with one or more hydroxy, M is an alkali metal or $NH_4^+$, and p is 1.
In some embodiments, R is independently selected from $-CH = CH_2$, $-CH(OH)CH_3$ and $-COCH_3$, M is an alkali metal or $NH_4^+$, and p is 1.
In some embodiments, R is independently selected from $-CH=CH_2$ and $-CH(OH)CH_3$, M is an alkali metal or $NH_4^+$, and p is 1.
In some embodiments, M is an alkali metal.
In some embodiments, M is Na or K.
In some embodiments, the porphyrin dimer salt bound with an ether linkage is selected from the group consisting of: bis [1- [6, 7-sodium dipropionate-1, 3, 5, 8-tetramethyl-4-vinyl-2-porphine] ethyl] ether, bis [1- [6, 7-sodium dipropionate-1, 3, 5, 8-tetramethyl-2-vinyl-4-porphine] ethyl] ether, 1- [6, 7-sodium dipropionate-1, 3, 5, 8-tetramethyl-2-vinyl-4-porphine] ethyl 1- [6', 7' -sodium dipropionate-1', 3', 5', 8' -tetramethyl-4-vinyl-2-porphine] ethyl ether, bis [1- [6, 7-potassium dipropionate-1, 3, 5, 8-tetramethyl-4-vinyl-2-porphine] ethyl] ether, bis [1- [6, 7-potassium dipropionate-1, 3, 5, 8-tetramethyl-2-vinyl-4-porphine] ethyl] ether, 1- [6, 7-potassium dipropionate-1, 3, 5, 8-tetramethyl-2-vinyl-4-porphine] ethyl 1- [6', 7' -potassium dipropionate-1', 3', 5', 8' -tetramethyl-4-vinyl-2-porphine] ethyl ether, bis [1- [6, 7-ammonium dipropionate-1, 3, 5, 8-tetramethyl-4-vinyl-2-porphine] ethyl] ether, bis [1- [6, 7-ammonium dipropionate-1, 3, 5, 8-tetramethyl-2-vinyl-4-porphine] ethyl] ether, and 1- [6, 7-ammonium dipropionate-1, 3, 5, 8-tetramethyl-2-vinyl-4-porphine] ethyl 1- [6', 7' -ammonium dipropionate-1', 3', 5', 8' -tetramethyl-4-vinyl-2-porphine] ethyl ether.
In some embodiments, the porphyrin dimer salt is sodium porphyrine (DVDMS), which is a sodium porphyrin dimer salt bound by an ether bond, and in some embodiments, the sodium porphyrine has a structure represented by formula (I), formula (II), or formula (III) above.

In some embodiments, the porphyrin dimer salt is bis [1- [6, 7-sodium dipropionate-1, 3, 5, 8-tetramethyl-4-vinyl-2-porphine] ethyl] ether (DVDMS-1), bis [1- [6, 7-sodium dipropionate-1, 3, 5, 8-tetramethyl-2-vinyl-4-porphine] ethyl] ether (DVDMS-2), 1- [6, 7-sodium dipropionate-1, 3, 5, 8-tetramethyl-2-vinyl-4-porphine] ethyl 1- [6', 7' -sodium dipropionate-1', 3', 5', 8' -tetramethyl-4-vinyl-2-porphine] ethyl ether (DVDMS-3) with the chemical structures shown below, or a mixture of the three (DVDMS-M).

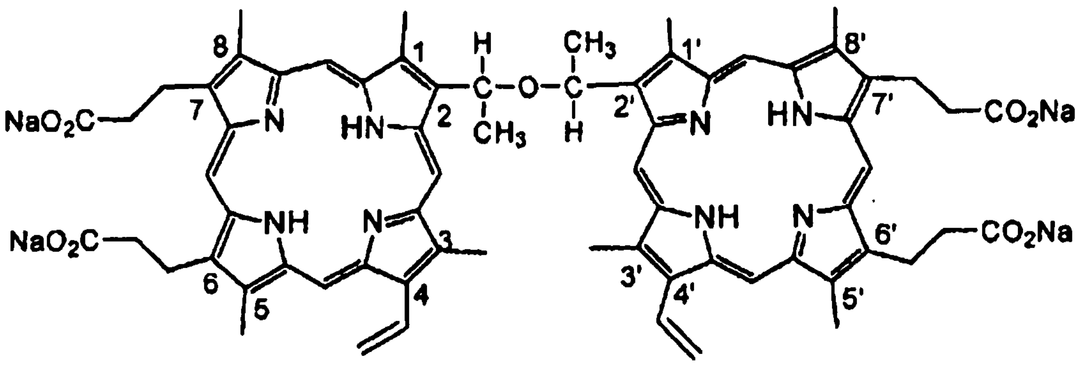

DVDMS-1

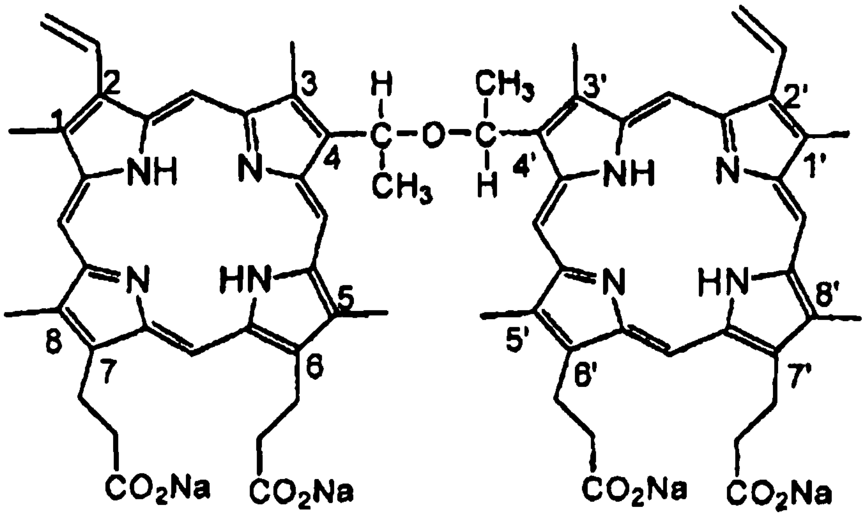

DVDMS-2

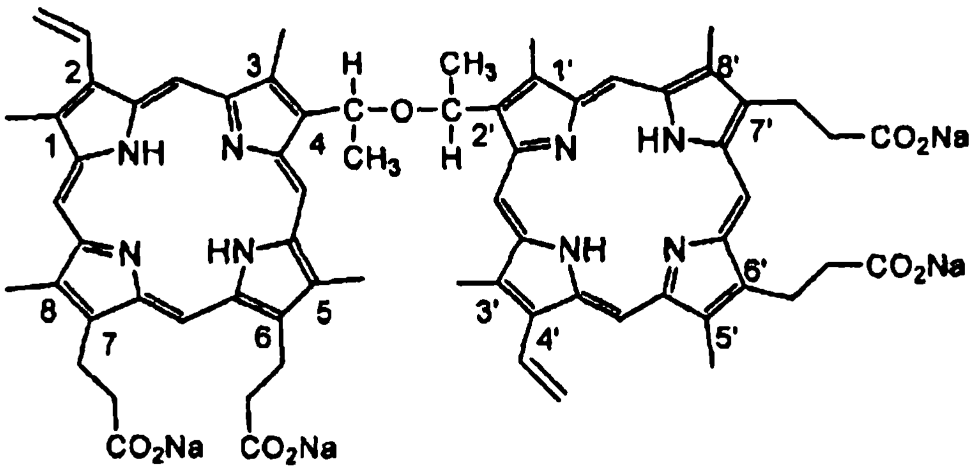

DVDMS-3

Further details regarding the photosensitizer comprising a porphyrin dimer salt are described in CN102030765B, the disclosure of which is incorporated herein in its entirety. In some embodiments, the photosensitizers of the present invention are used to practice the disclosed methods of treatment.

Combined therapy

The present application provides a combined therapy of photodynamic therapy based on a porphyrin dimer salt-based photosensitizer and immunological therapy for use in the treatment of cancer in an individual, comprising administering to said individual a therapeutically effective amount of porphyrin dimer salt photosensitizer in combination with an immune checkpoint inhibitor.

In the present application, the term "combined therapy" means that when two or more drugs are used in combination, they may be administered simultaneously, within an overlapping period of time, or sequentially. The administration routes of the two drugs administered in combination may be the same or different.

In the present application, the term "treatment" means alleviation of one or more symptoms associated with a disease, delay or avoid progression or worsening of a disease, or cure of a disease. For cancer (e.g., esophageal cancer), treatment may include curbing the development of the cancer, reducing its severity, slowing its growth, inhibiting its metastasis, and reducing recurrence, etc. But treatment does not require that the disease or symptoms associated therewith be completely eliminated.

In the present application, the term "individual" means an animal, including mammals and non-mammals. Mammal means any member of the mammalian class, including, but not limited to, humans, non-human primates (such as chimpanzees and other apes and monkeys), farm animals (such as cows, horses, sheep, goats, and pigs), domestic animals (such as rabbits, dogs, and cats), laboratory animals (including rodents, such as rats, mice, and guinea pigs), etc. Examples of non-mammals include, but are not limited to, birds, etc.

In the present application, the term "therapeutically effective amount" means that amount of a compound of the application or combined therapy which produces the biological or medical response sought or desired by a researcher or clinician.

In the present application, the photosensitizer described herein may be formulated in any suitable form and may be administered by any suitable route, may be formulated as a solution, suspension, emulsion, lyophilized formulation, etc. for injection (e.g., intra-arterial, intravenous, intramuscular, subcutaneous, intraperitoneal injection, etc.) or infusion administration, formulated as a tablet, solution, capsule, etc. for oral administration, formulated as ointment, cream, suppository, patch, etc. for topical administration, formulated as aerosol, spray, powder, etc. for inhalation administration. Preferred administration methods are generally injection infusion, oral and topical administration. Administration by injection/infusion allows the compounds of the application to reach distribution equilibrium rapidly, for example within 24 hours.

In some embodiments, the porphyrin dimer salt is administered in the range of 0.01-5mg/kg body weight. For example, 0.05-4mg/kg body weight, 0.1-2mg/kg body weight, or 0.2-1 mg/kg body weight.

In some embodiments, the individual is photodynamic treated with a laser some time after the administration of porphyrin dimer salt. Preferably, photodynamic therapy is performed on the individual with a laser 24-48 hours, especially 24 hours after the porphyrin dimer salt administration. In some embodiments, the laser has a wavelength of 631 ± 3nm,

such as 630nm, 631nm, or 632 nm. In some embodiments, the laser dosage is 60-300J/$cm^2$ , 70-300J/$cm^2$ , 80-300J/$cm^2$ , 90-300J/$cm^2$ ,90-290J/$cm^2$ , 90-280J/$cm^2$ , 90-270J/$cm^2$ , 90-260J/$cm^2$ , 90-250J/$cm^2$, 90-240J/$cm^2$ , 90-230J/$cm^2$ , 90-220J/$cm^2$ , 90-210J/$cm^2$ , 90-200J/$cm^2$ , 90-190J/$cm^2$ , 90-180J/$cm^2$ , 90-170J/$cm^2$ , 90-160J/$cm^2$ , 90-150J/$cm^2$ , 90-140J/$cm^2$ , 90-130J/$cm^2$ , 90-120J/$cm^2$ , 100-120J/$cm^2$, 100-110J/$cm^2$, preferably 90-300J/$cm^2$. In some embodiments, the laser is administered at a dose of 200J/$cm^2$ for advanced esophageal cancer that has caused near-complete obstruction.

In the present application, the "immune checkpoint inhibitor" refers to a drug capable of targeting an immune checkpoint molecule, releasing the inhibition of immune cells by tumor cells, causing immune cells to reactivate and recognize/kill cancer cells.

In some embodiments, the immune checkpoint inhibitor is PD-1 antagonist (PD-1 antibody) or PD-L1 antagonist (PD-L1 antibody).

In some embodiments, the PD-1 antibody includes, but is not limited to: Pembrolizumab, Nivolumab, Cemiplimab, Toripalimab, Sintilimab, Camrelizumab, Tislelizumab, Penpulimab, Sepalimab, Serplulimab and Putelimumab.

In some embodiments, the PD-L1 antibody includes, but is not limited to: Atezolizumab, Avelumab, Durvalumab, Envafolimab and Sugemalimab.

In some embodiments, the immune checkpoint inhibitor is administered to the individual after a period of time following administration of the photodynamic therapy. In some embodiments, the immune checkpoint inhibitor is administered to the individual after 20 to 50 hours, particularly 24 to 48 hours, of the laser administration. In some embodiments, combination treatment of DVDMS-PDT with PD-1 antibody can significantly reduce the rate of tumor growth in a mouse model with significantly enhanced anti-tumor effects compared to a DVDMS-PDT monotherapy regimen. In some embodiments, PD-1 antibody treatment between 24 hours and 48 hours after DVDMS-PDT has a superior antitumor effect with a tumor inhibition rate of over 98%.

In some embodiments, the porphyrin dimer salt is DVDMS-1, DVDMS-2, DVDMS-3, or DVDMS-M, and the immune checkpoint inhibitor is PD-1 antibody, e.g., Pembrolizumab.

## Examples

Materials:

1. Reagent and instrument

1.1 test Agents

Sodium porphyrine DVDMS-2 for injection (Shanghai Guangsheng Pharmaceutical Co., Ltd.); Pembrolizumab (purchased from Merck & Co., Inc.).

1.2 Illumination device

630nm semiconductor laser therapeutic device (XINGDA PDT 630II), laser wavelength meter (THORLABS), laser power meter (PM310D), equipped with Micro lens optical fiber.

2. Tumor cell strain

Human esophageal cancer Eca-109 cell strain, purchased from China Center for Type Culture Collection (CCTCC) in Wuhan, China.

3. Experimental animals

nu/nu nude mice, 18-22 g in weight, female, provided by Shanghai SLAC Laboratory Animal Co,Ltd..

### Example 1. Efficacy of combined therapy of sodium porphyrine-mediated photodynamic therapy (DVDMS-PDT) with PD-1 antibody in a mouse model of human esophageal cancer Eca-109

Purpose: observe the growth-inhibiting effect of DVDMS-PDT combined with PD-1 antibody (Pembrolizumab) on human esophageal cancer Eca-109 nude mouse xenograft tumor, optimize the time window between DVDMS-PDT and PD-1 antibody treatment, and provide a reference for clinical application.

Scheme:

1. Preparation of human esophageal cancer model

Harvest human esophageal cancer Eca-109 cells at logarithmic growth phase and dilute with culture medium to a cell concentration of $5*10^7$ cells/mL, and inoculate subcutaneously at the right thigh root of nude mice at an injection of 0.1mL per nude mouse. And 7-10 days later, grow the subcutaneous tumor at the root of the thigh on the right side of the nude mouse until the diameter is about 5-8 mm for later use.

2. Study of the effect of PD-1 antibody treatment at different time windows after DVDMS-PDT on tumor growth

Select qualified tumor-bearing mouse models as described above, measure the length and the width of the tumors using a vernier caliper, and stratify and group the mice according to tumor size, with 6 mice in each group. Prepare the sodium porphyrine DVDMS-2 for injection 24 hours before the experiment, and refrigerate in the dark before use. Administer DVDMS-2 via tail vein at a dose of 2mg/kg body weight (Day-1), followed by PDT treatment 24 hours later (Day 0). When performing tumor phototherapy, first adjust the 630nm semiconductor laser to lock the output power at the fiber end at 250mW, then position the optical fiber with the microlens at the end towards the target area, ensuring the laser is projected perpendicularly and completely covers the tumor, maintaining a spot diameter of 10mm, and the dosage of the treatment laser is about $70J/cm^2$.

Administer PD-1 antibody (Pembrolizumab) some time after the completion of DVDMS-PDT, and perform PD-1 antibody treatment on each group at time intervals shown in table 1, respectively, according to the experimental groups.

After the combination treatment, observe the growth of the tumor and the toxic reaction condition of the animal, wherein except the tumor illumination, the mice are strictly shielded from light for 3 days after being administrated from the tail vein, and then are raised with weak light. Kill the animal on the 21st day (Day 21) after receiving DVDMS-PDT, strip the tumors, weigh the tumors, evaluate the tumor inhibition effect of each group, and calculate the tumor growth inhibition rate of the drug, the evaluation index is relative tumor proliferation rate T/C (%), and the calculation formula is as follows:

$$T/C\ (\%) = (\text{Treatment group (T) RTV}/\text{Negative control group (C) RTV}) \times 100\%.$$

TABLE 1.

| Group | Treatment | Tumor weight | Tumor growth inhibition rate(%) |
|---|---|---|---|
| Negative control group | Blank, no treatment | 1.28±0.58 | 0 |
| Treatment group 1 | Only received DVDMS-PDT | 0.43±0.58** | 66.8 |
| Treatment group 2 | PD-1 antibody treatment administered on the same day as DVDMS-PDT (within 24 hours) | 0.36±0.13** | 71.9 |
| Treatment group 3 | PD-1 antibody treatment administered 24 hours after DVDMS-PDT | 0.01±0.04** | 99.4 |
| | | | |
| Treatment group 4 | PD-1 antibody treatment administered 48 hours after DVDMS-PDT | 0.02±0.04** | 98.7 |
| Treatment group 5 | PD-1 antibody treatment administered more than 48 hours (51 hours) after DVDMS-PDT | 0.33±0.15 | 74.2 |
| Photosensitizer DVDMS-PDT dosage: 2mg/kg body weight; laser dosage: 70J/cm2;<br>PD-1 antibody dosage: 20mg/kg body weight | | | |

Conclusion: as shown in table 1, the combined treatment of DVDMS-PDT with PD-1 antibody can significantly reduce the tumor growth rate in mouse models with significantly enhanced anti-tumor effect compared to the DVDMS-PDT monotherapy regimen. And 24-48 hours after receiving DVDMS-PDT is a preferable window period for immunological therapy. The results show that the treatment effect after less than 24 hours, or more than 48 hours after receiving DVDMS-PDT is better than that of the control group. In addition, the effect of PD-1 antibody treatment between 24 hours and 48 hours after DVDMS-PDT is more excellent, and the tumor inhibition rate can reach more than 98%. In addition, no

significant toxic response was observed in any of the groups treated.

Example 2. Therapeutic Effect of combined therapy of DVDMS-PDT with PD-1 antibody in clinical cases of advanced esophageal cancer

The patient, 80 years old, has advanced esophageal cancer, diagnosed due to dysphagia, pathological diagnosis revealed esophageal squamous cell carcinoma, high-grade intraepithelial neoplasia and focal infiltration, with lymph nodes and distant metastasis, and the pathological stage is T4N3M1. With six cycles of systemic chemotherapy (paclitaxel + cisplatin), the disease still develops significant progress, and the esophagus has approached total obstruction and is unable to eat, according to esophageal cancer treatment guidelines, there are no recommended treatment options, the expected survival is three to six months.

In order to improve the esophageal obstruction of the patient, thereby improving the eating condition of the patient and improving the life quality of the patient, researchers applied the combined therapy of DVDMS-PDT and PD-1 antibody (Pembrolizumab) to the patient.

Scheme:

The patient was administered with 0.2mg/kg body weight of sodium porphyrin DVDMS-2, and PDT treatment was performed 24 hours later at a treatment wavelength of 630 $\pm$ 3 nm and a treatment dose of the light of 200J/cm$^2$. The patient was given an immunological therapy with a dose of 200mg each time of Pembrolizumab administered 24 hours after PDT treatment. The administration of the Pembrolizumab was then continued at a frequency of once every three weeks.

After one week of the combined therapy of DVDMS-PDT and PD-1 antibody, the patient's esophageal obstruction was greatly relieved, and the patient was able to consume semi-liquid foods. The patient received six consecutive administrations of Pembrolizumab and the disease remained stable. The patient later discontinued treatment for personal reasons. The long-term follow-up for four years showed the patient was still alive.

Conclusion: the combination of DVDMS-PDT and PD-1 antibody has the systemic effect on controlling disease progression, inhibiting tumor recurrence and spread, and improving the eating condition of the patients with advanced esophageal cancer, especially patients with terminal esophageal cancer, is beneficial to the continuation of the life cycle of patients and improves the life quality of patients.

Example 3. Clinical trials of combined therapy of DVDMS-PDT and immunotherapeutic drugs

Target: patients aged 18 or older, histologically diagnosed as esophageal squamous cell carcinoma (ESCC), imageologically diagnosed as metastatic esophageal cancer, and failed at least one-line metastatic systemic therapy.

Scheme: patients meeting the above criteria received intravenous injection of sodium porphyrin DVDMS-2 (Day-1), followed by PDT treatment of ESCC primary site lesion 24 hours after DVDMS-2 administration (Day 0), immunological therapy was then intiated 24 hours after DVDMS-PDT with a fixed dose 200 mg of intravenous injection of PD-1 antibody (e.g., Pembrolizumab or other selected PD-1 antibody or PD-L1 antibody) (Day 1), followed by immunological therapy once every 21 days (1 cycle of immunological therapy every 21 days) for 8 cycles.

On Day 30 after receiving DVDMS-PDT (Day 30), esophagogastropic (OGD) examination was performed to evaluate the efficacy of the treatment of the primary site lesion and to determine whether it is necessary to receive DVDMS-PDT treatment again.

Expected results:

1. The ESCC primary site receiving DVDMS-PDT will generate new antigen, enhance the effect of immunological therapy, enhance the inhibition to tumor growth, metastasis and recurrence;
2. The combined therapy of DVDMS-PDT and immunological therapy drugs can enhance the efficacy of immunological therapy on primary site lesion and metastatic lesion, improve objective remission rate (ORR) and duration of remission (DOR);
3. The combined therapy of DVDMS-PDT and immunological therapy drugs can improve the quality of life (QoL) of ESCC patients;
4. The combined therapy of DVDMS-PDT and immunological therapy drugs can extend progression-free survival (PFS) of ESCC patients;
5. The combined therapy of DVDMS-PDT and immunological therapy drugs can extend overall survival (OS) of ESCC

patients.

Although this application has been specifically shown and described with reference to specific embodiments (some of which are preferred embodiments), it will be understood by those skilled in the art that various changes in form and detail may be made therein without departing from the spirit and scope of the application disclosed herein.

## Claims

1. A method of treating cancer in an individual, comprising administering to the individual a therapeutically effective amount of a photosensitizer comprising a porphyrin dimer salt, the photosensitizer is administered in combination with an immune checkpoint inhibitor, optionally wherein the cancer is esophageal cancer.

2. The method of claim 1, wherein the esophageal cancer is selected from the group consisting of: esophageal squamous cell carcinoma (ESCC), adenocarcinoma, mucoepidermoid carcinoma, small cell carcinoma, large cell carcinoma, neuroendocrine tumor, adenosquamous carcinoma, and undifferentiated carcinoma, optionally said esophageal cancer is esophageal squamous cell carcinoma (ESCC) or adenocarcinoma.

3. The method of claim 1, wherein the esophageal cancer is advanced esophageal cancer, or optionally, locally advanced esophageal cancer.

4. The method of claim 1, wherein the esophageal cancer is recurrent esophageal cancer, optionally local recurrent esophageal cancer, and/or metastatic esophageal cancer, optionally distant metastatic esophageal cancer.

5. The method of any one of claims 1-4, wherein the esophageal cancer exhibits resistance to at least one chemotherapy selected from the group consisting of: taxane-based antitumor chemotherapy drug, fluorouracil-based drug, or platinum-based drug.

6. The method of claim 5, wherein the taxane-based antitumor chemotherapy drug is selected from the group consisting of: paclitaxel or docetaxel.

7. The method of claim 5, wherein the fluorouracil-based drug is selected from the group consisting of: 5-fluorouracil, tegafur or capecitabine.

8. The method of claim 5, wherein the platinum-based drug is selected from the group consisting of: cisplatin, carboplatin, or oxaliplatin.

9. The method of any one of claims 1-4, wherein the esophageal cancer exhibits immunotherapeutic resistance.

10. The method of any of the preceding claims, wherein the individual has previously received radiotherapy and/or chemotherapy.

11. The method of any of the preceding claims, wherein the individual has been treated for a primary lesion and/or metastatic lesion.

12. The method of any of the preceding claims, wherein the porphyrin dimer salt has a structure represented by formula (I), formula (II), or formula (III):

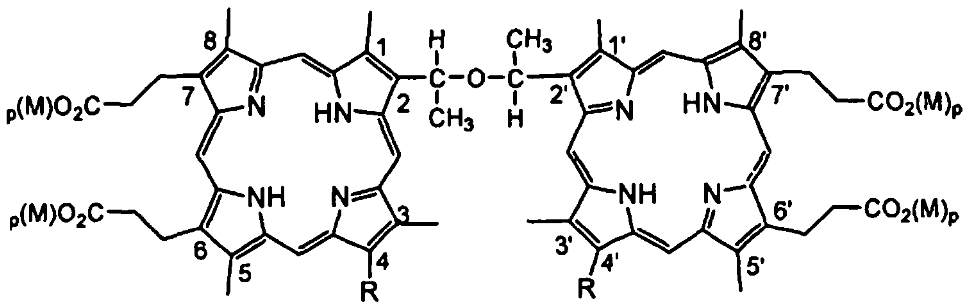

formula (I),

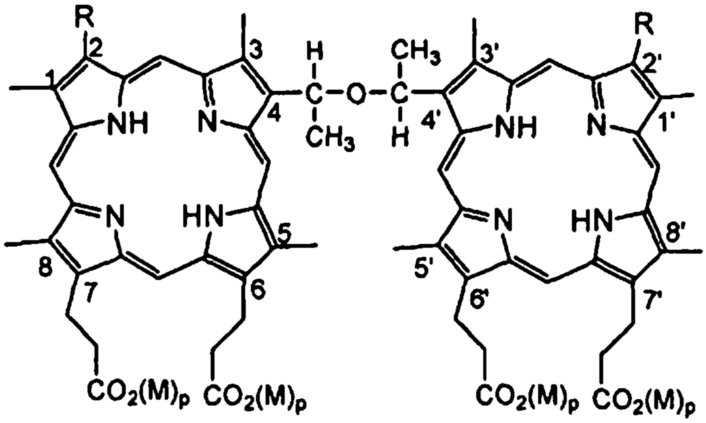

formula (II), or

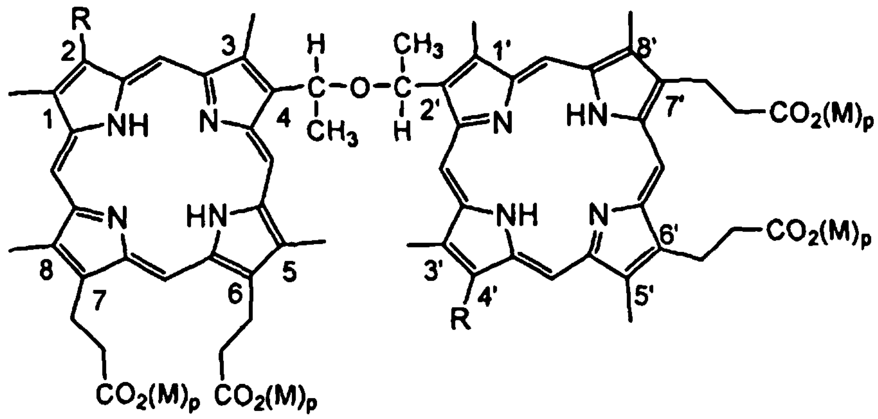

formula (III)

Wherein,

R is independently selected from $C_{1-6}$ alkyl, $C_{2-6}$ alkenyl, $C_{2-6}$ alkynyl, $C_{1-6}$ alkoxy and $C_{1-6}$ acyl, optionally substituted with one or more substituents selected from halogen and hydroxy,
M is an alkali metal, an alkaline earth metal or $NH_4^+$,
p is the reciprocal of the valence number of M.

**13.** The method of claim 12, wherein R is independently selected from $C_{1-6}$ alkyl, $C_{2-6}$ alkenyl, $C_{2-6}$ alkynyl, and $C_{1-6}$ acyl optionally substituted with one or more hydroxy groups, M is an alkali metal or $NH_4^+$, and p is 1.

**14.** The method of claim 13, wherein R is independently selected from $-CH = CH_2$, $- CH(OH)CH_3$ and $-COCH_3$, M is an

alkali metal or $NH_4^+$, and p is 1.

**15.** The method of claim 14, wherein R is independently selected from $-CH=CH_2$ and $-CH(OH)CH_3$, M is an alkali metal or $NH_4^+$, and p is 1.

**16.** The method of any one of claims 12 to 15, wherein M is an alkali metal.

**17.** The method of claim 16, wherein M is Na or K.

**18.** The method of claim 12, wherein the ether-bond bound porphyrin dimer salt is selected from the group consisting of:

bis [1- [6, 7-sodium dipropionate-1, 3, 5, 8-tetramethyl-4-vinyl-2-porphine] ethyl] ether,
bis [1- [6, 7-sodium dipropionate-1, 3, 5, 8-tetramethyl-2-vinyl-4-porphine] ethyl] ether,
1- [6, 7-sodium dipropionate-1, 3, 5, 8-tetramethyl-2-vinyl-4-porphine] ethyl 1- [6', 7' - sodium dipropionate-1', 3', 5', 8' -tetramethyl-4-vinyl-2-porphine] ethyl ether,
bis [1- [6, 7-potassium dipropionate-1, 3, 5, 8-tetramethyl-4-vinyl-2-porphine] ethyl] ether,
bis [1- [6, 7-potassium dipropionate-1, 3, 5, 8-tetramethyl-2-vinyl-4-porphine] ethyl] ether,
1- [6, 7-potassium dipropionate-1, 3, 5, 8-tetramethyl-2-vinyl-4-porphine] ethyl 1- [6', 7' - potassium dipropionate-1', 3', 5', 8' -tetramethyl-4-vinyl-2-porphine] ethyl ether,
bis [1- [6, 7-ammonium dipropionate-1, 3, 5, 8-tetramethyl-4-vinyl-2-porphine] ethyl] ether,
bis [1- [6, 7-ammonium dipropionate-1, 3, 5, 8-tetramethyl-2-vinyl-4-porphine] ethyl] ether, and
1- [6, 7-ammonium dipropionate-1, 3, 5, 8-tetramethyl-2-vinyl-4-porphine] ethyl 1- [6', 7' -ammonium dipropionate-1', 3', 5', 8' -tetramethyl-4-vinyl-2-porphine] ethyl ether.

**19.** The method of any one of claims 12-18, wherein the porphyrin dimer salt is administered in the range of 0.01-5mg/kg body weight.

**20.** The method of any of the preceding claims, wherein the individual is photodynamic treated using a laser 24-48 hours after the porphyrin dimer salt administration.

**21.** The method of claim 20, wherein the laser has a wavelength of 631 $\pm$ 3nm, such as 630nm, 631nm or 632 nm.

**22.** The method of claim 20 or 21, wherein the dose of the laser is 90-300J/$cm^2$ .

**23.** The method of any of the preceding claims, wherein the immune checkpoint inhibitor is administered to the individual after a period of time following the laser administration.

**24.** The method of claim 23, wherein the immune checkpoint inhibitor is administered to the individual 20 to 50 hours (optionally 24 to 48 hours) after the laser administration.

**25.** The method of claim 23 or 24, wherein the immune checkpoint inhibitor is selected from the group consisting of: PD-1 antagonist and PD-L1 antagonist, wherein the PD-1 antagonist is optionally PD-1 antibody, the PD-L1 antagonist is optionally PD-L1 antibody.

**26.** The method of claim 25, wherein the PD-1 antibody comprises Pembrolizumab, Nivolumab, Cemiplimab, Toripalimab, Sintilimab, Camrelizumab, Tislelizumab, Penpulimab, Sepalimab, Serplulimab, and Putelimumab.

**27.** The method of claim 25, wherein the PD-L1 antibody comprises Atezolizumab, Avelumab, Durvalumab, Envafolimab and Sugemalimab.

**28.** The method of any of the preceding claims, wherein the porphyrin dimer salt is DVDMS-1, DVDMS-2, DVDMS-3, or DVDMS-M, and wherein the immune checkpoint inhibitor is Pembrolizumab.

**29.** Use of a photosensitizer comprising the porphyrin dimer salt as defined in any one of claims 12 to 18 in the preparation of drug for the treatment of esophageal cancer in an individual, wherein the treatment comprises the combined therapy of a photosensitizer and an immune checkpoint inhibitor, optionally wherein the cancer or the individual is as defined in any one of claims 1-11.

**30.** A photosensitizer suitable for use in treating cancer in an individual, wherein the photosensitizer comprises the porphyrin dimer salt as defined in any one of claims 12 to 18, and the photosensitizer is administered in combination with an immune checkpoint inhibitor.

**31.** The photosensitizer of claim 30, wherein the immune checkpoint inhibitor is as defined in claims 25 to 27.

**32.** The photosensitizer of claim 30 or 31, wherein the porphyrin dimer salt is administered in the range of 0.01-5mg/kg body weight.

**33.** The photosensitizer of any one of claims 30-32, wherein the individual is photodynamic treated with a laser 24 hours after the administration of the porphyrin dimer salt.

**34.** The photosensitizer of any one of claims 30-33, wherein the laser has a wavelength of 631 ± 3nm, such as 630nm, 631nm or 632 nm.

**35.** The photosensitizer of any one of claims 33-34, wherein the dose of the laser is 90-300J/$cm^2$.

**36.** The photosensitizer of any one of claims 33-35, wherein the immune checkpoint inhibitor is administered to the individual after a period of time following the laser administration.

**37.** The photosensitizer of any one of claims 33-36, wherein the immune checkpoint inhibitor is administered to the individual 20 to 50 hours (optionally 24 to 48 hours) after the laser administration.

**38.** The photosensitizer of any one of claims 30-37, wherein the cancer or the individual is as defined in claims 1-11.

## INTERNATIONAL SEARCH REPORT

International application No.
**PCT/CN2024/093175**

### A. CLASSIFICATION OF SUBJECT MATTER

C07D519/00(2006.01)i; A61K31/409(2006.01)i; A61K41/00(2020.01)i; A61K39/395(2006.01)i; A61P35/00(2006.01)i

According to International Patent Classification (IPC) or to both national classification and IPC

### B. FIELDS SEARCHED

Minimum documentation searched (classification system followed by classification symbols)

IPC: A61K, C07D, A61P

Documentation searched other than minimum documentation to the extent that such documents are included in the fields searched

Electronic data base consulted during the international search (name of data base and, where practicable, search terms used)

CNTXT, ENTXTC, DWPI, VEN, CNKI, STN, 万方, WANFANG, 百度学术, Baidu Scholar, Science Direct: 光声制药, 卟啉二聚体, DVDMS, 华卟啉钠, 免疫检查点抑制剂, PD-1, PD-L1, 帕博利珠单抗, 光敏剂, 光动力治疗, 食管癌, photodynamic therapy, PDT, photosensitizer, esophageal cancer

### C. DOCUMENTS CONSIDERED TO BE RELEVANT

| Category* | Citation of document, with indication, where appropriate, of the relevant passages | Relevant to claim No. |
|---|---|---|
| X | CN 102030765 A (QINGLONG HI-TECH CO., LTD.) 27 April 2011 (2011-04-27) claims 1-7 and 18-19 | 29-38 |
| Y | CN 102030765 A (QINGLONG HI-TECH CO., LTD.) 27 April 2011 (2011-04-27) claims 1-7 and 18-19 | 1-28 |
| Y | CN 109381428 A (FUDAN UNIVERSITY) 26 February 2019 (2019-02-26) claims 1 and 5 | 1-28 |
| X | ZHANG, Y.F. et al. "Biomimetic Nanoemulsion for Synergistic Photodynamic-Immunotherapy Against Hypoxic Breast Tumor" *Angewandte Chemie International Edition*, Vol. vol. 60, 18 March 2021 (2021-03-18), pp. 10647-10653 | 1-38 |
| Y | KR 10-2019-0103999 A (NAT UNIV. PUSAN IND. UNIV. COOP. FOUND.) 05 September 2019 (2019-09-05) claims 1, 5 and 16 | 1-28 |
| A | CN 105582541 A (ZHANG, Shaoliang) 18 May 2016 (2016-05-18) claims 1-21 | 1-38 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 August 2024** | **16 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/093175**

**C. DOCUMENTS CONSIDERED TO BE RELEVANT**

| Category* | Citation of document, with indication, where appropriate, of the relevant passages | Relevant to claim No. |
|---|---|---|
| A | CN 105396135 A (QINGLONG HI-TECH CO., LTD.) 16 March 2016 (2016-03-16) claims 1-10 | 1-38 |

Form PCT/ISA/210 (second sheet) (July 2022)

**INTERNATIONAL SEARCH REPORT**

International application No.
**PCT/CN2024/093175**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-28**
because they relate to subject matter not required to be searched by this Authority, namely:

Claims 1-28 relate to a method for treating cancer in an individual, which falls within subject matter for which no search is required by the International Searching Authority as defined in PCT Rule 39.1(iv). A search is carried out on the basis that the designation of the subject matter of said claims is a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/093175**

| Patent document cited in search report | Publication date (day/month/year) | Patent family member(s) | Publication date (day/month/year) |
|---|---|---|---|
| CN 102030765 A | 27 April 2011 | None | |
| CN 109381428 A | 26 February 2019 | None | |
| KR 10-2019-0103999 A | 05 September 2019 | KR 102245552 B1 | 29 April 2021 |
| CN 105582541 A | 18 May 2016 | None | |
| CN 105396135 A | 16 March 2016 | None | |

Form PCT/ISA/210 (patent family annex) (July 2022)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description

- CN 102030765 B